Europäisches Patentamt

(19) European Patent Office

Office européen des brevets

(11) Veröffentlichungsnummer: **0 185 171**
**B1**

## (12) EUROPÄISCHE PATENTSCHRIFT

(45) Veröffentlichungstag der Patentschrift:
07.10.87

(51) Int. Cl.⁴: **C 07 C 21/08, C 07 C 17/34, C 07 C 17/38**

(21) Anmeldenummer: 85113565.7

(22) Anmeldetag: 25.10.85

(54) **Verfahren zur Herstellung von Vinylidenchlorid.**

(30) Priorität: 14.11.84 DE 3441548

(43) Veröffentlichungstag der Anmeldung:
25.06.86 Patentblatt 86/26

(45) Bekanntmachung des Hinweises auf die Patenterteilung:
07.10.87 Patentblatt 87/41

(84) Benannte Vertragsstaaten:
BE DE FR IT NL

(56) Entgegenhaltungen:
DE - B - 1 230 418

(73) Patentinhaber: BASF Aktiengesellschaft,
Carl-Bosch-Strasse 38, D-6700 Ludwigshafen (DE)

(72) Erfinder: Krome, Gerd, Dr., Am Wingertsberg 28,
D-6719 Weisenheim (DE)
Erfinder: Pradel, Guenter, Am Egelsee 23, D-6720 Speyer
(DE)

## Beschreibung

Die Erfindung betrifft ein Verfahren zur Herstellung von Vinylidenchlorid durch Abspaltung von Chlorwasserstoff aus 1,1,2-Trichlorethan in wässeriger alkalischer Emulsion bei erhöhtem Druck und erhöhter Temperatur, Entspannen des Reaktionsgutes und Trennen der wässerigen Phase von der gasförmigen Phase und destillative Aufarbeitung der gasförmigen Phase.

Aus der DE-PS 1 230 418 ist ein Verfahren zur kontinuierlichen Herstellung von Vinylidenchlorid durch Umsetzen von 1,1,2-Trichlorethan in wässeriger alkalischer Emulsion unter Abspalten von Chlorwasserstoff bekannt, wobei das Trichlorethan in der wässerigen alkalischen Emulsion derart verteilt ist, dass sich eine Teilchengrösse zwischen 5 und 20 µm ausbildet und die Umsetzung bei einer Temperatur durchgeführt wird, die oberhalb des Siedepunktes Vinylidenchlorid unter Atmosphärendruck liegt und bei einem Druck, bei dem das Vinylidenchlorid in der Reaktionszone in flüssiger Phase vorliegt. In dieser Patentschrift werden auch die bis dahin bekannten Verfahren abgehandelt, wobei nach dem Verfahren der Patentschrift eine wesentlich bessere Raum-Zeit-Ausbeute erzielt wird als nach den bislang bekannten Verfahren. Vinylidenchlorid wird hauptsächlich zu wässerigen Polymerdispersionen verarbeitet, die zum Flammfestausrüsten von Fasern und Beschichten von Verpackungsfolien für Lebens- und Arzneimittel dienen. Demnach wird an die Reinheit des Ausgangsmaterials eine hohe Anforderung gestellt, dass praktisch frei von toxischen Nebenprodukten sein muss.

Es hat sich gezeigt, dass nach bekannten Verfahren hergestelltes Vinylidenchlorid mitunter noch geringe Mengen an störenden Verunreinigungen enthielt. Ausserdem sind die bei dem bekannten Verfahren erzielten Raum-Zeit-Ausbeuten mitunter noch nicht befriedigend.

Der Erfindung liegt die Aufgabe zugrunde, ein Verfahren zur Herstellung von Vinylidenchlorid der oben beschriebenen Art zu schaffen, das es ermöglicht, ein Vinylidenchlorid mit besonderem hohen Reinheitsgrad herzustellen, wobei die Raum-Zeit-Ausbeute des Verfahrens gegenüber dem bekannten Verfahren wesentlich verbessert ist.

Die Aufgabe der Erfindung wird durch ein Verfahren zur Herstellung von Vinylidenchlorid gelöst, bei dem man das Abspalten von Chlorwasserstoff aus 1,1,2-Trichlorethan in wässeriger alkalischer Emulsion bei erhöhten Drucken und erhöhten Temperaturen, Entspannen des Reaktionsgutes und Trennen der wässerigen Phase von der gasförmigen Phase und destillative Aufarbeitung der gasförmigen Phase vornimmt. Das Verfahren ist dadurch gekennzeichnet, dass man die Abspaltung bei Drucken zwischen 6 und 10 bar und bei Temperaturen im Bereich von 76 bis 80°C durchführt, das Reaktionsgut entspannt und einen Teil der flüssigen Phase in den Reaktionsraum zurückführt und die destillative Aufarbeitung in der gasförmigen Phase bei Überdrucken in einem Bereich von 100 bis 300 mm Wassersäule vornimmt.

Es hat sich gezeigt, dass man nach dem erfindungsgemässen Verfahren ein Reaktionsprodukt erhält, dass mehr als 99,9 Gew.-% Vinylidenchlorid enthält. Im Reaktionsprodukt sind cis-Dichlorethylen, 1,1-Dichlorethan, 1,1,2-Trichlorethan sowie Monochloracetylen nicht nachweisbar. Der Gehalt an Ethylchlorid liegt unter 200 ppm der an Vinylchlorid unter 10 ppm und der an trans-Dichlorethylen unter 20 ppm. Die Raum-Zeit-Ausbeute beträgt etwa 200 kg Vinylidenchlorid/l Reaktionsraumvolumen und Tag.

Zur Abspaltung von Chlorwasserstoff aus dem 1,1,2-Trichlorethan werden eine wässerige Lösung von Alkali oder Erdalkalihydroxiden, die vorzugsweise 2 bis 0,2 Gew.-% des Hydroxides enthält, mit dem Trichlorethan und einem Teil der Flüssigphase des entspannten Reaktionsgutes gemischt. Die Mischung erfolgt in einer Pumpe, vorzugsweise einer Kreiselpumpe, die bewirkt, dass die Ausgangsstoffe nach dem Austritt aus der Pumpe in Emulsion vorliegen. Die Tröpfchen des emulgierten 1,1,2-Trichlorethans haben einen Teilchendurchmesser, der im Bereich von < 10 µm liegt. Die Emulsion enthält das 1,1,2-Trichlorethan in Gewichtsmengen, die 0,5 bis 2,5 Gew.-% der Emulsion ausmachen. Die Komponenten, d.h. die wässerige Phase des abgetrennten Reaktionsgutes und die frisch zudosierte wässerige Lauge wird in einem Verhältnis gemischt, das in der wässerigen Phase zwischen 0,2 und 2 Gew.-%, vorzugsweise 0,4 bis 1 Gew.-% freie Lauge vorhanden ist. Vorzugsweise arbeitet man mit Natronlauge.

Das als Emulsion vorliegende Reaktionsgut wird nun in eine Reaktionszone eingebracht, in der dafür gesorgt wird, dass das Reaktionsgut auf Temperaturen von 76 bis 80°C gehalten wird. Die strenge Einhaltung dieses Temperaturbereiches ist wichtig, da sonst nicht gewährleistet ist, dass man ein Reaktionsprodukt mit hohem Reinheitsgrad erhält und die Anlage ohne Störung über längere Zeit betrieben werden kann. Die Reaktionszone hat eine Längenausdehnung, die das 500- bis 1200fache des Durchmessers der Querschnittsfläche der Reaktionszone ausmacht. In der Reaktionszone herrscht ein Druck von 6 bis 10 bar. Um das Entmischen der Emulsion zu vermeiden, wird dieses Reaktionsgut mit hoher Geschwindigkeit durch die Reaktionszone geführt, so dass eine Turbulenz herrscht. Die Verweilzeit des Reaktionsgutes in der Reaktionszone beträgt zwischen 0,05 und 0,25, vorzugsweise 0,1 bis 0,2 Minuten. Das Reaktionsgut wird dann über ein Ventil in ein Entspannungsgefäss entspannt, so dass eine Auftrennung der wässerigen Flüssigphase und der gasförmigen Phase der Reaktionsprodukte entsteht. In dem Entspannungsgefäss herrscht ein Druck, der auch in der anschliessenden Kolonne zur destillativen Aufarbeitung der gasförmigen Phase der Reaktionsproduktes herrscht. Mitunter ist es zweckmässig, zwischen Entspannungsgefäss und Destillationskolonne ein Abscheidungsgefäss anzuordnen, in dem eventuell übertretende Flüssigphasenanteile von der gasförmigen Phase getrennt und mit dem in dem Entspannungsgefäss anfallenden Flüssigphase vereint werden. Von dieser Flüssigphase wird ein Teil über eine Pumpe mit den Ausgangsstoffen wie oben erwähnt, gemischt und in die Reaktionszone zurückgeführt. Der verbleibende Anteil der Flüssigphase wird der Wasserdampfdestillationsaufarbeitung zugeführt. Dieser weitere Anteil entspricht in seiner Menge der Menge an wässeriger Phase, die das Alkali- oder Erdalkalihydroxid enthält und die mit dem 1,1,2-Trichlorethan gemischt wird. Die Anteile der flüssigen Phase des Reaktionsgutes, die in das Reaktionsgefäss zurückgeführt werden, wird so gewählt, dass eine vorgegebene Temperatur in der Destillationskolonne eingehalten werden kann.

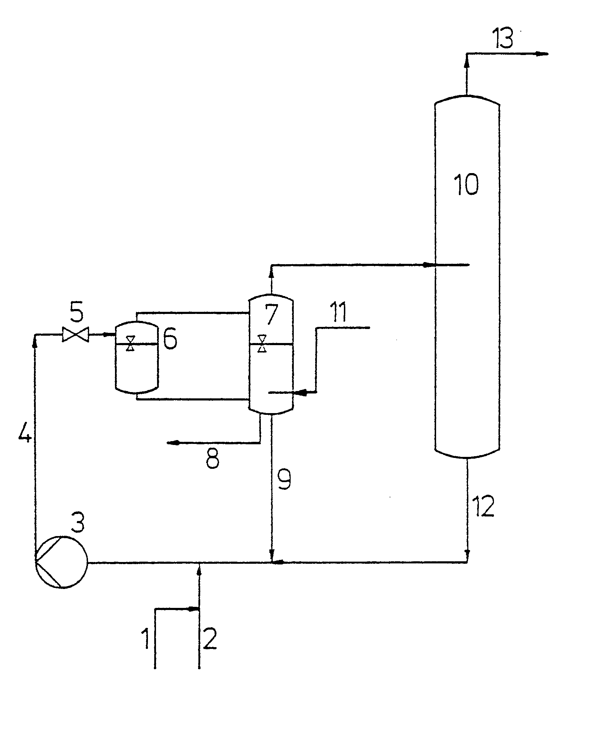

Eine besonders vorteilhafte Ausführung des Verfahrens kann in einer Apparatur vorgenommen werden, die in der Figur schematisch wiedergegeben ist. Die Reaktanten, d.h. das 1,1,2-Trichlorethan (1) und die wässerige Alkalihydroxidlösung (2) werden gemischt und über eine Kreiselpumpe (3) mit dem aus dem Abscheider (7) stammenden Anteil der Flüssigphase des Reaktionsgutes und dem aus dem Sumpf der Destillationskolonne (10) stammenden Anteil, der durch die Leitung (9) und (12) zugeführt wird zu einer Emulsion gemischt und in den Rohrreaktor (4) übergeführt. Es baut sich ein Druck im Reaktor auf, der dem Reaktionsdruck entspricht. Das Reaktionsgut wird über ein Ventil (5) in das Entspannungsgefäss (6) entspannt, worin eine Aufteilung der Flüssigphase und der gasförmigen Phase des Reaktionsgutes erfolgt. Die gasförmige Phase tritt in das Abscheidergefäss (7) über, worin eine weitere Abtrennung der flüssigen Bestandteile erfolgt. Abscheidergefäss (7) ist über eine Leitung mit der Flüssigphase des Entspannungsgefässes verbunden. Der Abscheider wird mit Wasserdampf (11) beheizt, so dass sich im gesamten Reaktionssystem d.h. in der Reaktionszone in den Entspanungs- und Abscheidebehältern die Reaktionstemperatur ausbildet. Die gasförmige Phase des Reaktionsgutes tritt in die Destillationskolonne (10) über. Das reine Reaktionsprodukt wird über die Leitung (13) entnommen und der Sumpf über die Leitung (12) dem aus dem Abscheider austretende Flüssigphase (9) vereint und mit den Reaktanten (1) und (2) der Pumpe (3) zugeführt.

Es ist ein besonderer Vorteil des erfindungsgemässen Verfahrens, dass eine Anlage entsprechend dem Verfahren über einen längeren Zeitraum (der Erfahrungszeitraum beträgt 4 Jahre), betrieben werden kann ohne dass eine Reinigung der Kolonne oder anderen Reaktionsgefässen erforderlich ist. Nach dem bekannten Verfahren war es erforderlich, Füllkörper in der Reaktionszone sowie in der Destillationskolonne in einem Zeitraum von Monaten auszuwechseln.

## Beispiel 1:

Es wird in einer in der Abbildung dargestellten Apparatur gearbeitet. Es werden über (1) je Stunde 10,6 t 1,1,2-Trichlorethan und über (2) in Form von 25%iger Lösung 3,4 t NaOH (= 100%ig) eingebracht. In der Pumpe (3) werden die Reaktanten zusammen mit dem aus dem Abscheider (7) über die Leitung (9) mit der Leitung (12) stammenden wässerigen Phase sowie dem Sumpf der Destillation vermischt. Es wird ein Druck von 10 bar aufgebaut. Die Emulsion durchläuft den Reaktor (4) mit einer Geschwindigkeit von 5 m/sec. Über das Ventil (5) wird die Reaktionslösung auf einen Überdruck von 150 mm Wassersäule entspannt, der sich in dem Entspannungsgefäss (6) im Abscheider (7) und in der Kolonne (10) ausbildet. Hier stellt sich am Kopf der Kolonne (10) eine Siedetemperatur des Vinylidenchlorid von 33°C ein. Es werden am Kopf der Kolonne (10) 7,5 t Vinylidenchlorid pro Stunde abgezogen. Das Rücklaufverhältnis in der Kolonne beträgt 1:5. Von der in dem Entspannungsgefäss und im Abscheider sich absetzenden Flüssigphase werden 3,5 t der Pumpe (3) zugefahren. Ein Anteil von 14 t wird über (8) der weiteren Auarbeitung durch Wasserdampfdestillation zugeführt. Durch Dampfheizung (11) wird der Abscheider (7) auf einer Temperatur von 78°C gehalten, bis sich infolge der freiwerdenden Reaktionswärme über den Reaktor (4) und die Entspannungsgefässe (6) sowie den Abscheider (7) hält.

Das Vinylidenchlorid hat folgende Zusammensetzung, was gaschromatographisch ermittelt wurde.

| | |
|---|---|
| Vinylidenchlorid | 99,9 Gew.-% |
| Ethylchlorid | 200 ppm |
| Vinylchlorid | 10 ppm |
| trans-Dichlorethylen | 20 ppm |
| cis-Dichlorethylen | < 1 ppm |
| 1,1-Dichlorethan | < 1 ppm |
| 1,1,2-Trichlorethan | < 1 ppm |
| Monochloracetylen | < 1 ppm |
| Dichloracetylen | < 10 ppm |

Das über Leitung (9) abgeführte Flüssigphasengemisch enthält 0,1 Gew.-% Natronlauge und 15 Gew.-% Natriumchlorid und ist frei von Chlorkohlenwasserstoffen.

Die Raum-Zeit-Ausbeute beträgt 200 kg Vinylidenchlorid je Liter Reaktionsvolumen und Tag.

## Beispiel 2 (Vergleich):

Man arbeitet wie in Beispiel 1 angegeben in der dort beschriebenen Apparatur und hält die Temperatur im Abscheider dem Entspannungsgefäss und im Reaktionsrohr auf 70°C.

Hierdurch verringert sich die Reaktionsgeschwindigkeit, so dass ein grosser Teil des nichtumgesetzten 1,1,2-Trichlorethan zusammen mit der Flüssigphase wieder in das Reaktionsrohr zurück im Kreis geführt wird. Die Ausbeute bezogen auf NaOH verschlechtert sich um etwa 20%, die Raum-Zeit-Ausbeute ist stark verringert, sie beträgt <100 kg/l. Hierdurch steigt der Energiebedarf, so dass diese Arbeitsweise als nicht wirtschaftlich angesehen werden kann.

## Beispiel 3 (Vergleich):

Man arbeitet wie in Beispiel 1 angegeben und stellt die Temperatur im Reaktionsrohr im Entspannungsgefäss und im Abscheider auf 85°C ein. Nun ist die Raum-Zeit-Ausbeute befriedigend. Sie beträgt >1400 kg/l. Es verschlechtert sich jedoch die Ausbeute hinsichtlich 1,1-Dichlorethan. Die Selektivität des Umsatzes von 1,1,2-Trichlorethan zum Vinylidenchlorid beträgt 92%. Es werden hier also etwa 8% des 1,1,2-Trichlorethans zu anderen chlorierten Kohlenwasserstoffen hauptsächlich zu cis- und trans-Dichlorethylen umgesetzt.

*Beispiel 4 (Vergleich):*

Es wird wie in Beispiel 1 angegeben gearbeitet und lediglich der Druck in der Destillationskolonne (10) auf 5 m Wassersäule gehalten. Hierdurch stellt sich eine Temperatur am Kopf der Kolonne von 41°C ein. Bereits nach 10 Tagen hatten sich Polymerisatablagerungen gebildet, so dass die Füllkörper der Kolonne ausgetauscht werden mussten.

### Patentanspruch

Verfahren zur Herstellung von Vinylidenchlorid durch Abspaltung von Chlorwasserstoff aus 1,1,2-Trichlorethan in wässeriger alkalischer Emulsion bei erhöhtem Druck und erhöhter Temperatur, Entspannen des Reaktionsgutes und Trennen der wässerigen Phase von der gasförmigen Phase und destillative Aufarbeitung der gasförmigen Phase, dadurch gekennzeichnet, dass man die Abspaltung bei Drucken zwischen 6 und 10 bar und bei einer Temperatur im Bereich von 76 bis 80°C durchführt, das Reaktionsgut entspannt und einen Teil der Flüssigphase in den Reaktionsraum zurückführt und die destillative Aufarbeitung der gasförmigen Phase bei Überdrucken in einem Bereich von 100 bis 300 mm Wassersäule vornimmt.

### Claim

A process for the preparation of vinylidene chloride by eliminating hydrogen chloride from 1,1,2-trichloroethane in aqueous alkaline emulsion under superatmospheric pressure and at elevated temperature, releasing the pressure on the reacted mixture, separating the aqueous phase from the gaseous phase and working up the latter by distillation, wherein the elimination reaction is carried out under from 6 to 10 bar and at from 76 to 80°C, the pressure on the reacted mixture is released and some of the liquid phase is recycled to the reaction space, and the working up of the gaseous phase by distillation is carried out under superatmospheric pressure of from 100 to 300 mm water column.

### Revendication

Procédé de préparation du chlorure de vinylidène par séparation d'acide chlorhydrique à partir de 1,1,2-trichloréthane, en émulsion aqueuse alcaline, à température élevée et sous pression élevée, détente du produit de la réaction et séparation de la phase aqueuse de la phase gazeuse et traitement par distillation de la phase gazeuse, caractérisé en ce que l'on réalise la séparation à une température qui varie de 76 à 80°C sous des pressions qui fluctuent de 6 à 10 bars, on détend le produit de la réaction et on renvoie une partie de la phase liquide dans l'enceinte de réaction et on entreprend le traitement par distillation de la phase gazeuse sous des surpressions qui varient dans la gamme de 100 à 300 mm de colonne d'eau.